# EUROPEAN PATENT APPLICATION

(11) **EP 4 643 828 A1**
(43) Date of publication of application: **05.11.2025**
(21) Application number: 23910149.6
(22) Date of filing: 13.12.2023
(51) Int. Cl.: A61F 2/966

(54) **CONVEYOR**

(30) Priority: 30.12.2022 CN 202211730472
(71) Applicant: Lifetech Scientific (Shenzhen) Co., Ltd., Shenzhen, Guangdong 518063 (CN)
(72) Inventor: LV, Cheng, Shenzhen, Guangdong 518063 (CN); LIU, Kui, Shenzhen, Guangdong 518063 (CN)
(74) Representative: Michalski Hüttermann & Partner Patentanwälte mbB
(86) International application number: PCT/CN2023/138480
(87) International publication number: WO 2024/140219

(57) **Abstract**

A delivery device (100), including a sheath core assembly (10) and a delivery handle (20). The sheath core assembly (10) includes a sheath tube (11) and a sheath tube connector (12) connected to a proximal end of the sheath tube (11). The delivery handle (20) includes a housing (21), a screw rod (22) and a toothed block (23) located in the housing (21), and a locking device connected to the toothed block (23). The screw rod (22) includes an upper screw rod (221a) and a lower screw rod (221b) which are oppositely arranged in a radial direction. The two sides of the upper screw rod (221a) and the lower screw rod (221b) are each provided with a spacing groove (22a) extending in an axial direction. The proximal end of the sheath tube (11) extends into the screw rod (22) of the delivery handle (20), and two sides of the sheath tube connector (12) in the radial direction extend out from the spacing grooves (22a) on the two sides respectively. At least two toothed blocks (23) are provided. The at least two toothed blocks (23) are arranged at intervals in a circumferential direction of the screw rod (22), and all the toothed blocks (23) are not opposite to all the spacing grooves (22a) at the same time. According to the delivery device, a plurality of toothed blocks are not opposite to the spacing grooves of the screw rod at the same time, so that an effective meshing area of the toothed block and the screw rod is effectively ensured, thereby ensuring stable use of the instrument.

## Description

### Technical Field

The present invention relates to the technical field of interventional medical devices, and particularly, to a delivery device.

### Background Art

With the advancement of technology, minimally invasive surgery has become increasingly popular in treating various diseases. In minimally invasive surgery, a medical device (such as a stent) is implanted into the human body through a delivery device via an organ or blood vessel.

During interventional clinical surgery, a surgeon may occasionally encounter an embarrassing situation while operating, positioning, and releasing a device on an operating table. For example, in the reconstruction of left subclavian artery (LSA), the descending aorta typically presents with dissection or aneurysm; and in such cases, a stent is delivered through a delivery device to the aortic arch via a peripheral blood vessel of various levels such as femoral artery, iliac artery, abdominal aorta, thoracic aorta, brachial artery, and axillary artery. The human blood vessels have complex and tortuous anatomical structures, and cause great resistance to the stent when the stent is released. During slow release of the stent, limitations on the number, arrangement, and size of toothed blocks reduce an interaction surface of a duct with the toothed block when the toothed block passes through a gap of the duct, so that the toothed block is susceptible to sliding during slow release of the stent. This will lead to failure of slow release and seriously affect the operation of the surgeon.

### Summary of the Invention

In view of this, the present invention proposes a delivery device, to at least solve the problem of sliding of a toothed block.

To achieve this objective, the present invention adopts the following technical solutions.

The present invention provides a delivery device, which includes a sheath core assembly and a delivery handle. The sheath core assembly includes a sheath tube and a sheath tube connector connected to a proximal end of the sheath tube. The delivery handle includes a housing, a screw rod and a toothed block located in the housing, and a locking device connected to the toothed block. The screw rod includes an upper screw rod and a lower screw rod which are oppositely arranged in a radial direction. Two sides of the upper screw rod and the lower screw rod are each provided with a spacing groove extending in an axial direction. The proximal end of the sheath tube extends into the screw rod of the delivery handle, and two sides of the sheath tube connector in the radial direction extend out from the spacing grooves on the two sides respectively. At least two toothed blocks are provided. The at least two toothed blocks are arranged at intervals in a circumferential direction of the screw rod. The toothed block is either in a first state of meshing with an external thread outside the screw rod or in a second state of separating from the external thread outside the screw rod. All the toothed blocks are not opposite to all the spacing grooves at the same time. The locking device is used to keep the toothed block in the first state of meshing without the action of external force, or to keep the toothed block in the second state of separating under the action of external force.

In an embodiment, three toothed blocks are provided, and the three toothed blocks are arranged at intervals in the circumferential direction of the screw rod, so that at least one toothed block is always not opposite to the spacing grooves.

In an embodiment, the three toothed blocks are distributed at equal angles in the circumferential direction, so that at least two toothed blocks are always not opposite to the spacing grooves.

In an embodiment, when axial positions of the three toothed blocks are the same, the external thread on the screw rod adopts a three-thread structure; or when the axial positions of the three toothed blocks are not exactly the same or completely different, the external thread on the screw rod adopts a double-thread structure or single-thread structure.

In an embodiment, the locking device includes a screw rod sleeve sleeved on the screw rod and a trigger sleeve sleeved on the screw rod; a first through hole is formed in a side wall close to a distal side of the screw rod sleeve and is used for a distal end of the toothed block to extend into, and a second through hole is formed in a side wall of the trigger sleeve and is used for a proximal end of the toothed block to extend into; the toothed block is placed between the screw rod sleeve and the trigger sleeve; when the toothed block is in the first state, the distal end of the toothed block passes through the first through hole and engages with the external thread on the screw rod, and the proximal end of the toothed block is suspended relative to the distal end and extends into the second through hole; and when the toothed block is in the second state, the trigger sleeve is retreated toward the proximal end under the action of external force, the retreated trigger sleeve presses the proximal end of the toothed block such that the distal end of the toothed block is tilted and suspended, and the toothed block is separated from the screw rod.

In an embodiment, the locking device further includes a spring top sleeve axially arranged relative to a proximal end of the screw rod sleeve, and a proximal end surface of the spring top sleeve radially extends out to form an annular blocking surface; and the locking device further includes a return spring sleeved on the screw rod sleeve and the spring top sleeve, one end of the return spring abuts against a proximal end surface of the trigger sleeve, the other end of the return spring abuts against the blocking surface, and the return spring is used to provide elastic force such that the retreated trigger sleeve is returned to the initial first state.

In an embodiment, a connecting protrusion is convexly arranged on an outer wall of the trigger sleeve, a through hole is formed in the housing, and the locking device further includes a trigger; the connecting protrusion passes through the through hole and is secured to the trigger; and under the action of external force, the trigger drives the trigger sleeve to retreat axially.

In an embodiment, the sheath core assembly further includes an inner sheath core inserted into the sheath tube, a tip head is arranged at a distal end of the inner sheath core, and a blocking platform is convexly arranged on an inner wall of the housing; at least two blocking platforms are arranged at intervals in the axial direction, and the sheath tube connector extending out from the spacing grooves on the two sides is placed between the two blocking platforms; the delivery device further includes an elastic member, a proximal end of the elastic member is secured to the housing, and a distal end of the elastic member abuts against a proximal end of the sheath tube connector; before the delivery device is treated, a distal end surface of the sheath tube abuts against the tip head of the inner sheath core, a distal end of the sheath tube connector is a certain distance away from the blocking platform at the distal end, and the elastic member is in a compressed state; and after the delivery device is treated, the elastic member pushes the sheath tube connector toward the distal end until a distal end surface of the sheath tube connector abuts against the tip head again.

In an embodiment, the elastic member is sleeved on the screw rod, the proximal end of the elastic member abuts against the blocking platform at the proximal end, and the distal end of the elastic member abuts against a proximal end surface of the sheath tube connector extending out from the spacing grooves.

In an embodiment, the delivery device further includes a stop block, and a limit block is convexly arranged on the inner wall of the housing; two limit blocks are arranged at intervals in the axial direction, and the stop block is axially limited between the two limit blocks; the stop block passes through the spacing groove and extends into the screw rod; and the elastic member is placed in the screw rod, the proximal end of the elastic member placed in the screw rod abuts against the stop block, and the distal end of the elastic member abuts against the proximal end surface of the sheath tube connector.

According to the delivery device, a plurality of toothed blocks are not opposite to the spacing grooves of the screw rod at the same time, so that an effective meshing area of the toothed block and the screw rod is effectively ensured, thereby ensuring stable use of the instrument. In addition, the elastic member is arranged on the proximal side of the sheath tube connector, and the sheath tube connector can move toward the distal end under the action of elastic force provided by the elastic member, thereby compensating for the shortening of the sheath tube connector due to treatment and preventing the head from having a step difference when the sheath tube is shortened.

### Brief Description of the Drawings

FIG. 1 is schematic diagram of a partially exploded structure of a delivery device according to an exemplary embodiment of the present invention;
FIG. 2 is a schematic diagram of a partial connection between a toothed block and a screw rod in a delivery device according to an exemplary embodiment of the present invention;
FIG. 3 is a schematic cross-sectional diagram of a delivery device according to exemplary embodiment of the present invention;
FIG. 4 is a schematic diagram of a partial structure of a plurality of toothed blocks in a same axial position in a delivery device according to an exemplary embodiment of the present invention;
FIG. 5 is a schematic diagram of a partial structure of a plurality of toothed blocks in different axial positions in a delivery device according to an exemplary embodiment of the present invention;
FIG. 6 is a schematic diagram of a partial semi-section of a delivery device according to an exemplary embodiment of the present invention;
FIG. 7 is a schematic diagram of the layout of a shortened sheath tube; and
FIG. 8 is a diagram of a partial section of another arrangement of an elastic member in a delivery device according to an exemplary embodiment of the present invention.

### Detailed Description of the Invention

To make the objectives, technical solution, and advantages of the present invention clearer, the following describes exemplary implementations of the present disclosure in detail with reference to the accompanying drawings. Although the exemplary implementations of the present disclosure are illustrated in the accompanying drawings, it is to be understood that the present disclosure may be implemented in various forms and is not limited to the implementations described herein. On the contrary, these implementations are provided to enable a more thorough understanding of the present disclosure and to fully convey the scope of the present disclosure to those skilled in the art.

It is to be understood that the terms used herein is for the purpose of describing particular exemplary implementations only and is not intended to be limiting. As used herein, the singular forms "a", "an", and "the" may be intended to include the plural forms as well, unless the context clearly indicates otherwise. The terms "include", "comprise", "contain", and "have" are inclusive, and thus specify the presence of stated features, steps, operations, elements, and/or components, but do not preclude the presence or addition of one or more other features, steps, operations, elements, components, and/or combinations thereof. The method steps, processes, and operations described herein are not to be construed as necessarily requiring their performance in the particular order described or illustrated, unless the order of execution is explicitly stated. It is also to be understood that additional or alternative steps may be employed.

Although the terms "first", "second", "third", and the like may be used herein to describe a plurality of elements, components, regions, layers and/or sections, these elements, components, regions, layers and/or sections should not be limited by these terms. These terms may be only used to distinguish one element, component, region, layer or section from another region, layer or section. The terms, such as "first" and "second", and other numerical terms when used herein do not imply a sequence or order unless the context clearly indicates otherwise. Therefore, a first element, component, region, layer or section discussed below could be termed a second element, component, region, layer or section without departing from the teachings of the exemplary implementations.

For ease of description, spatially relative relationship terms may be used herein to describe the relationship of one element or feature relative to another element or feature as shown in the figures. These relative relationship terms are, for example, "interior", "exterior", "inside", "outside", "beneath", "below", "on", and "above". The spatially relative relationship terms are intended to encompass different orientations of the device in use or operation in addition to the orientation depicted in the figures. For example, if the device in the figures is turned over, elements described as "beneath" or "below" other elements or features would then be oriented "on" or "above" the other elements or features. Thus, the exemplary term "below" may encompass both an orientation of above and below. The device may be otherwise oriented (rotated 90 degrees or at other orientations) and is accordingly interpreted by the spatially relative relationship descriptors used herein.

It is also to be noted that in the field of interventional medical devices, a proximal end refers to an end closer to an operator, and a distal end refers to an end away from the operator; a direction of the central axis of rotation of an object, such as a cylinder or a tube, is defined as an axial direction; a direction (perpendicular to both the axis and a cross-sectional radius) around the axis of an object, such as a cylinder or a tube, is defined as a circumferential direction; and a direction along a diameter or radius is defined as a radial direction. It is worth noting that the "end" appearing in the terms "proximal end", "distal end", "one end", "the other end", "first end", "second end", "initial end", "terminal end", "two ends", "free end", "upper end", "lower end", and the like is not limited to an end head, end point or end surface, but also includes a portion, extending an axial distance and/or radial distance from the end head, end point or end surface, of an element to which the end head, end point or end surface belongs. The above definitions are only for the convenience of description and should not be construed as limitations on the present utility model.

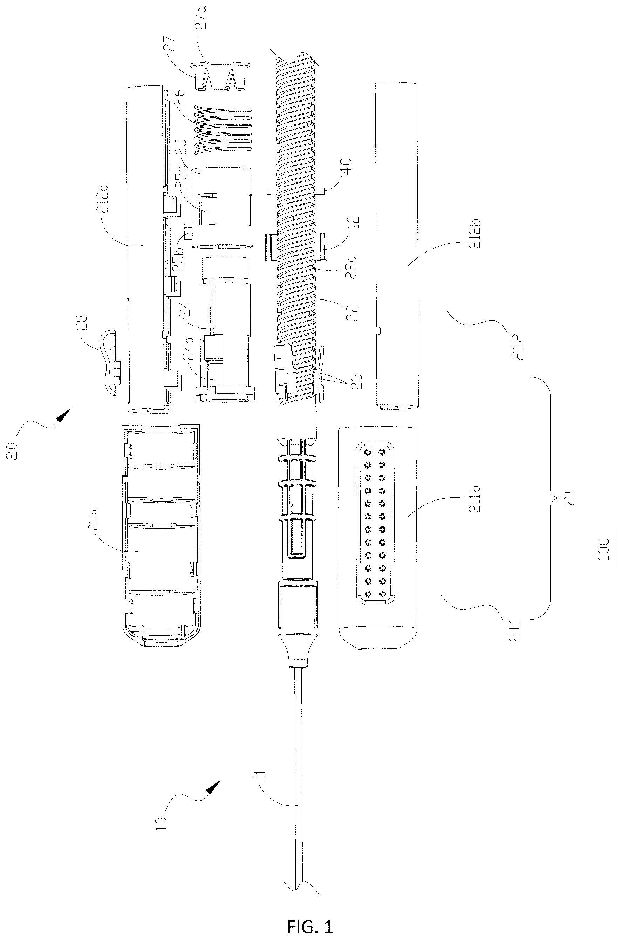

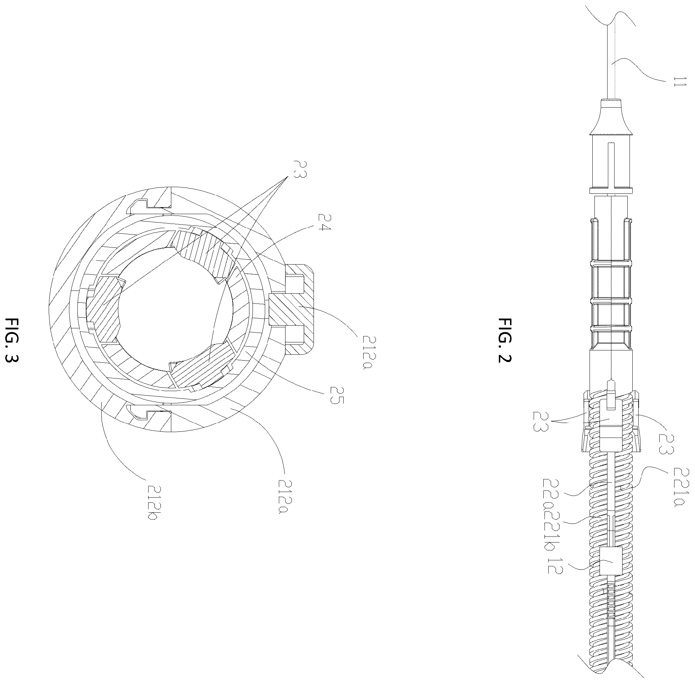

Referring to FIG. 1 and FIG. 2, in this aspect, a delivery device 100 is exemplarily provided. The delivery device 100 includes a sheath core assembly 10 and a delivery handle 20. The sheath core assembly 10 includes a sheath tube 11 and a sheath tube connector 12 connected to a proximal end of the sheath tube 11. The delivery handle 20 includes a housing 21, a screw rod 22 and a toothed block 23 located in the housing 21, and a locking device connected to the toothed block 23.

Referring to FIG. 1, the housing 21 includes a fixed handle 211 and a moving handle 212 which are oppositely arranged in an axial direction. The moving handle 212 is located on a proximal side. For ease of disassembly, the fixed handle 211 and the moving handle 212 are each of a structure having oppositely arranged upper and lower portions. As shown in FIG. 1, the fixed handle 211 includes an upper fixed portion 211a and a lower fixed portion 211b which are oppositely arranged, and the moving handle 212 includes an upper moving portion 212a and a lower moving portion 212b which are oppositely arranged. The screw rod 22 passes through the fixed handle 211 and the moving handle 212, and the toothed block 23 and the sheath tube connector 12 are located in the moving handle 212.

Referring to FIG. 1 and FIG. 2 again, the screw rod 22 includes an upper screw rod 221a and a lower screw rod 221b which are oppositely arranged in a radial direction. Two sides of the upper screw rod 221a and the lower screw rod 221b are each provided with a spacing groove 22a extending in the axial direction, so that the screw rod 22 has two symmetrically arranged spacing grooves 22a. The proximal end of the sheath tube 11 extends into the screw rod 22 of the delivery handle 20, and two sides of the sheath tube connector 12 in the radial direction extend out from the spacing grooves 22a on the two sides respectively. The sheath tube connector 12 is secured to the sheath tube 11, the sheath tube connector 12 may axially move along the spacing grooves 22a, and the sheath tube 11 axially moves as the sheath tube connector 12 moves.

At least two toothed blocks 23 are provided. The at least two toothed blocks 23 are arranged at intervals in a circumferential direction of the screw rod 22. The toothed block 23 is either in a first state of meshing with an external thread outside the screw rod 22 or in a second state of separating from the external thread outside the screw rod 22. All the toothed blocks 23 are not opposite to all the spacing grooves 22a at the same time. Through such an arrangement, at least one toothed block 23 is always not opposite to the spacing grooves 22a, thereby always effectively ensuring an effective meshing area of the toothed block 23 and the external thread of the screw rod 22, and avoiding the sliding of the toothed block. Generally, the two spacing grooves 22a are symmetrically arranged in the radial direction, that is, located on two sides. Therefore, if two toothed blocks 23a are provided, to prevent the two toothed blocks 23 from being opposite to the two spacing grooves at the same time, the two toothed blocks 23 are asymmetrically arranged. That is, when one toothed block 23 is opposite to the spacing grooves 22a, the other toothed block is necessarily not opposite to the spacing grooves.

Specifically, in this embodiment, as shown in FIG. 1 and FIG. 2, three toothed blocks 23 are provided. The three toothed blocks 23 are arranged at intervals in the circumferential direction of the screw rod 22, so that at least one toothed block 23 is always not opposite to the spacing grooves 22a. When three toothed blocks 23 are provided, angles between the three toothed blocks 23 may be set as needed, as long as at least one toothed block 23 is always not opposite to the spacing grooves 22a. In a preferred implementation, as shown in FIG. 3, to ensure rotation and stability of rotation, the three toothed blocks 23 are distributed at equal angles in the circumferential direction, so that at least two toothed blocks 23 are always not opposite to the spacing grooves 22a. That is, when one toothed block 23 is opposite to the symmetrically arranged spacing grooves 22a, the other two toothed blocks are necessarily not to opposite to the spacing grooves 22a. The two toothed blocks 23 are meshed with the external thread of the screw rod 22, thereby further increasing the meshing area, avoiding sliding of the toothed blocks, and enhancing the overall stability.

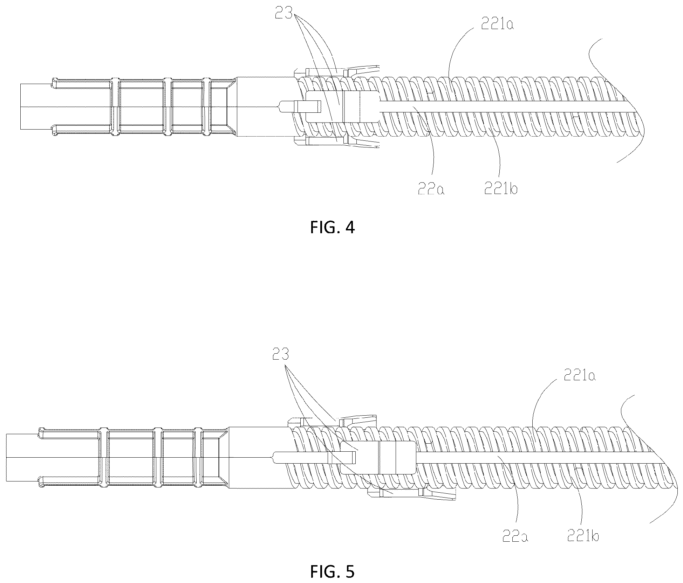

As shown in FIG. 4, when axial positions of the three toothed blocks 23 are the same, the external thread on the screw rod 22 adopts a three-thread structure. As shown in FIG. 5, when the axial positions of the three toothed blocks 23 are not exactly the same or completely different, the external thread on the screw rod 22 adopts a double-thread structure or single-thread structure.

The locking device is used to keep the toothed block 23 in the first state of meshing without the action of external force, or keep the toothed block 23 in the second state of separating under the action of external force.

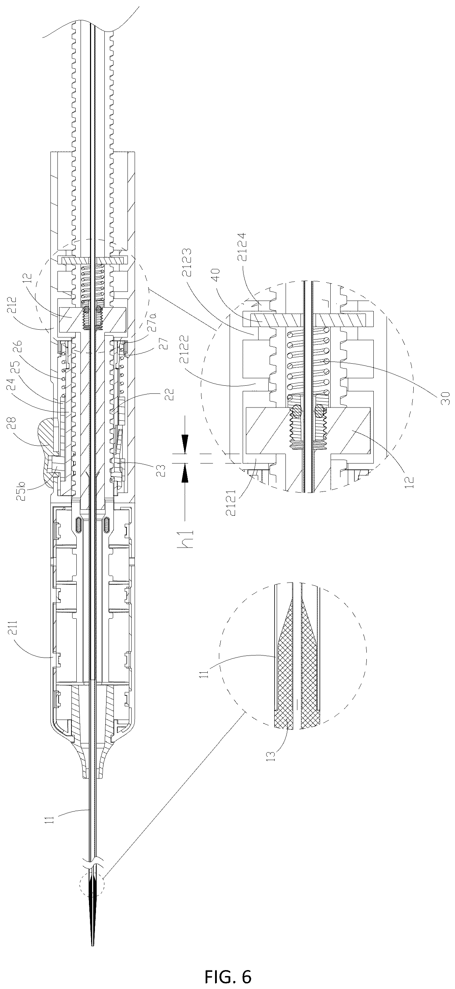

Specifically, as shown in FIG. 1 and FIG. 6, the locking device includes a screw rod sleeve 24 sleeved on the screw rod 22 and a trigger sleeve 25 sleeved on the screw rod sleeve 24. A first through hole 24a is formed in a side wall close to a distal side of the screw rod sleeve 24 and is used for a distal end of the toothed block 23 to extend into, and a second through hole 25a is formed in a side wall of the trigger sleeve 25 and is used for a proximal end of the toothed block 23 to extend into. The toothed block 23 is placed between the screw rod sleeve 24 and the trigger sleeve 25. When the toothed block is in the first state, the distal end of the toothed block 23 passes through the first through hold 24a and engages with the external thread on the screw rod 22, and the proximal end of the toothed block 23 is suspended relative to the distal end and extends into the second through hole. When the toothed block is in the second state, the trigger sleeve 25 is retreated toward the proximal end under the action of external force, the retreated trigger sleeve 25 presses the proximal end of the toothed block 23 such that the distal end of the toothed block 23 is tilted and suspended, and the toothed block is separated from the screw rod 22.

Further, the locking device further includes a spring top sleeve 27 axially arranged relative to a proximal end of the screw rod sleeve 24, and a proximal end surface of the spring top sleeve 27 radially extends out to form an annular blocking surface 27a. The locking device further includes a return spring 26 arranged on the screw rod sleeve 24 and around the spring top sleeve 27, one end of the return spring 26 abuts against a proximal end surface of the trigger sleeve 25, the other end of the return spring abuts against the blocking surface 27a, and the return spring 26 is used to provide elastic force such that the retreated trigger sleeve 25 is returned to the initial first state. For ease of assembly, a plurality of V-shaped grooves are concavely formed in a distal end surface of the spring top sleeve 27.

Referring to FIG. 6, the trigger sleeve 25 moves backward to drive the toothed block 23 away from the center. At this moment, the moving handle 121 may move linearly on the screw rod 22 without applying a force to the trigger sleeve 25. The return spring 26 applies a forward force to the trigger sleeve 25, and the toothed block 23 engages with the screw rod 22. At this moment, the moving handle 212 and the screw rod 22 can only rotate spirally but not linearly.

Referring to FIG. 1 and FIG. 6 again, to facilitate the application of external force to the trigger sleeve 25, a connecting protrusion 25b is convexly arranged on an outer wall of the trigger sleeve 25, a through hole (not shown) is formed in the housing 21, and the locking device further includes a trigger 28; the connecting protrusion 25b passes through the through hole and is secured to the trigger 28; and under the action of external force, the trigger 28 drives the trigger sleeve to retreat axially.

According to the delivery device, a plurality of toothed blocks are not opposite to the spacing grooves of the screw rod at the same time, so that an effective meshing area of the toothed block and the screw rod is effectively ensured, thereby ensuring stable use of the instrument.

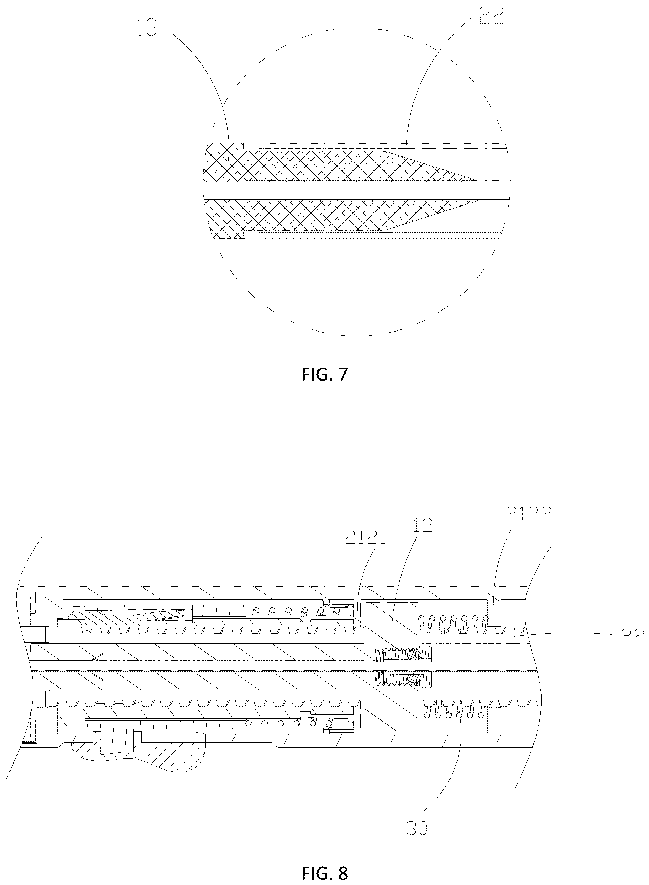

However, the main body of the sheath tube 11 is made of a polymer material. As shown in FIG. 7, during sterilization and storage, the material will shorten in length due to its own stress, forming a step difference between the proximal end of the sheath tube 11 and a tip head 13. In this case, if surgery is conducted, the head of the sheath tube 11 will scrape against a blood vessel and even scratches the blood vessel in severe cases.

In view of this, based on the above, the delivery device is further modified in the present invention. Referring to FIG. 6, the sheath core assembly 10 further includes an inner sheath core inserted into the sheath tube 11, a tip head 13 is arranged at a distal end of the inner sheath core, and a blocking platform is convexly arranged on an inner wall of the housing 21. At least two blocking platforms are arranged at intervals in the axial direction. The two blocking platforms are a blocking platform 2121 and a blocking platform 2122, respectively. The sheath tube connector 12 extending out from the spacing grooves 22a on the two sides is placed between the blocking platform 2121 and the blocking platform 2122. The delivery device 100 further includes an elastic member 30, a proximal end of the elastic member 30 is secured to the housing 21, and a distal end of the elastic member 30 abuts against a proximal end of the sheath tube connector 12.

Before the delivery device 100 is treated, the treatment involved in this embodiment refers to any treatment that will cause shortening of the sheath tube 11, such as sterilization. Referring to FIG. 6, before treatment, a distal end surface of the sheath tube 11 abuts against the tip head 13 of the inner sheath core, a distal end of the sheath tube connector 12 is a certain distance h1 away from the blocking platform at the distal end, the distance h1 is greater than zero, and the elastic member 30 is in a compressed state. The sheath tube 11 is resistant to bending when the elastic member 30 is in an elastic supporting state, that is, an elastic force provided by the elastic member 30 cannot cause the sheath tube 11 to bend. After the delivery device 100 is treated, the elastic member 30 pushes the sheath tube connector 12 toward the distal end until a distal end surface of the sheath tube connector 12 abuts against the tip head 13 again. At this moment, because the sheath tube connector 12 and the sheath tube 11 both move toward the distal end, h1 is reduced or h1 is reduced to zero.

Referring to FIG. 6 again, in an implementation, the proximal end of the elastic member 30 is secured to the housing 21. The delivery device 100 further includes a stop block 40, and a limit block is convexly arranged on the inner wall of the housing 21. Two limit blocks are arranged at intervals in the axial direction. The two limit blocks are a limit block 2123 and a limit block 2124, respectively. The stop block 40 is axially limited between the limit block 2123 and the limit block 2124. The stop block 40 passes through the spacing groove 22a and extends into the screw rod 22. The elastic member 30 is placed in the screw rod 22, the proximal end of the elastic member 30 placed in the screw rod 22 abuts against the stop block 40, and the distal end of the elastic member 30 abuts against a proximal end surface of the sheath tube connector 12.

Referring to FIG. 8, in another implementation, the proximal end of the elastic member 30 is secured to the housing 21. The elastic member 30 is inserted into the screw rod 22, the proximal end of the elastic member 30 abuts against the blocking platform 2122 at the proximal end, and the distal end of the elastic member 30 abuts against the proximal end surface of the sheath tube connector 12 extending out from the spacing grooves 22a.

In the further solution, the elastic member is arranged on the proximal side of the sheath tube connector, and the sheath tube connector can move toward the distal end under the action of elastic force provided by the elastic member, thereby compensating for the shortening of the sheath tube connector due to treatment and preventing the head from having a step difference when the sheath tube is shortened. In this embodiment, the structure for securing the sheath tube connector to the handle is designed as a movable structure, and the elastic member applies a certain elastic force to the sheath tube connector. Because the sheath tube at a stent is tightly fitted with the stent, the distal end of the sheath tube is secured by a certain friction. However, because the elastic member applies an elastic force to the sheath tube connector, and the sheath tube connector has a certain space for free movement, when the sheath tube tends to shorten, the sheath tube connector will move toward the proximal end, thereby avoiding shortening of the proximal end of the sheath tube. In addition, the scenario that the inner sheath core cannot be completely withdrawn into the sheath tube due to shortening of the sheath tube is also avoided.

The technical features in the foregoing embodiments may be combined in different ways to form new embodiments. For ease of description, not all possible combinations of the technical features in the foregoing embodiments are described. However, combinations of these technical features are considered to be within the scope of this description as long as there is no contradiction in the combinations.

The foregoing embodiments merely represent several implementations of the present invention, and the description thereof is relatively specific and detailed, but it should not be understood as limiting the patent scope of the present invention. It is to be pointed out that, for those skilled in the art, several modifications and improvements may be made without departing from the concept of the present invention, which all fall within the scope of protection of the present invention. Therefore, the scope of patent protection of the present invention shall be subject to the appended claims.

## Claims

1. A delivery device, comprising sheath core assembly and a delivery handle, wherein the sheath core assembly comprises a sheath tube and a sheath tube connector connected to a proximal end of the sheath tube, and the delivery handle comprises a housing, a screw rod and a toothed block located in the housing, and a locking device connected to the toothed block; the screw rod comprises an upper screw rod and a lower screw rod which are oppositely arranged in a radial direction, two sides of the upper screw rod and the lower screw rod are each provided with a spacing groove extending in an axial direction, the proximal end of the sheath tube extends into the screw rod of the delivery handle, and two sides of the sheath tube connector in the radial direction extend out from the spacing grooves on the two sides respectively; at least two toothed blocks are provided, the at least two toothed blocks are arranged at intervals in a circumferential direction of the screw rod, the toothed block is either in a first state of meshing with an external thread outside the screw rod or in a second state of separating from the external thread outside the screw rod, wherein all the toothed blocks are not opposite to all the spacing grooves at the same time; and the locking device is used to keep the toothed block in the first state of meshing without the action of external force, and keep the toothed block in the second state of separating under the action of external force.

2. The delivery device according to claim 1, wherein three toothed blocks are provided, and the three toothed blocks are arranged at intervals in the circumferential direction of the screw rod, so that at least one toothed block is always not opposite to the spacing grooves.

3. The delivery device according to claim 2, wherein the three toothed blocks are distributed at equal angles in the circumferential direction, so that at least two toothed blocks are always not opposite to the spacing grooves.

4. The delivery device according to claim 2 or 3, wherein when axial positions of the three toothed blocks are the same, the external thread on the screw rod adopts a three-thread structure; or when the axial positions of the three toothed blocks are not exactly the same or completely different, the external thread on the screw rod adopts a double-thread structure or single-thread structure.

5. The delivery device according to any one of claims 1 to 3, wherein the locking device comprises a screw rod sleeve sleeved on the screw rod and a trigger sleeve sleeved on the screw rod sleeve; a first through hole is formed in a side wall close to a distal side of the screw rod sleeve and is used for a distal end of the toothed block to extend into, and a second through hole is formed in a side wall of the trigger sleeve and is used for a proximal end of the toothed block to extend into; the toothed block is placed between the screw rod sleeve and the trigger sleeve; when the toothed block is in the first state, the distal end of the toothed block passes through the first through hole and engages with the external thread on the screw rod, and the proximal end of the toothed block is suspended relative to the distal end and extends into the second through hole; and when the toothed block is in the second state, the trigger sleeve is retreated toward the proximal end under the action of external force, the retreated trigger sleeve presses the proximal end of the toothed block such that the distal end of the toothed block is tilted and suspended, and the toothed block is separated from the screw rod.

6. The delivery device according to claim 5, wherein the locking device further comprises a spring top sleeve axially arranged relative to a proximal end of the screw rod sleeve, and a proximal end surface of the spring top sleeve radially extends out to form an annular blocking surface; and the locking device further comprises a return spring sleeved on the screw rod sleeve and the spring top sleeve, one end of the return spring abuts against a proximal end surface of the trigger sleeve, the other end of the return spring abuts against the blocking surface, and the return spring is used to provide elastic force such that the retreated trigger sleeve is returned to the initial first state.

7. The delivery device according to claim 5, wherein a connecting protrusion is convexly arranged on an outer wall of the trigger sleeve, a through hole is formed in the housing, and the locking device further comprises a trigger; the connecting protrusion passes through the through hole and is secured to the trigger; and under the action of external force, the trigger drives the trigger sleeve to retreat axially.

8. The delivery device according to any one of claims 1 to 3, wherein the sheath core assembly further comprises an inner sheath core inserted into the sheath tube, a tip head is arranged at a distal end of the inner sheath core, and a blocking platform is convexly arranged on an inner wall of the housing; at least two blocking platforms are arranged at intervals in the axial direction, and the sheath tube connector extending out from the spacing grooves on the two sides is placed between the two blocking platforms; the delivery device further comprises an elastic member, a proximal end of the elastic member is secured to the housing, and a distal end of the elastic member abuts against a proximal end of the sheath tube connector; before the delivery device is treated, a distal end surface of the sheath tube abuts against the tip head of the inner sheath core, a distal end of the sheath tube connector is a certain distance away from the blocking platform at the distal end, and the elastic member is in a compressed state; and after the delivery device is treated, the elastic member pushes the sheath tube connector toward the distal end until a distal end surface of the sheath tube connector abuts against the tip head again.

9. The convey according to claim 8, wherein the elastic member is sleeved on the screw rod, the proximal end of the elastic member abuts against the blocking platform at the proximal end, and the distal end of the elastic member abuts against a proximal end surface of the sheath tube connector extending out from the spacing grooves.

10. The delivery device according to claim 8, wherein the delivery device further comprises a stop block, and a limit block is convexly arranged on the inner wall of the housing; two limit blocks are arranged at intervals in the axial direction, and the stop block is axially limited between the two limit blocks; the stop block passes through the spacing groove and extends into the screw rod; and the elastic member is placed in the screw rod, the proximal end of the elastic member placed in the screw rod abuts against the stop block, and the distal end of the elastic member abuts against a proximal end surface of the sheath tube connector.
